(19) Europäisches Patentamt
European Patent Office
Office européen des brevets

(11) EP 1 741 704 A1

(12) EUROPEAN PATENT APPLICATION
published in accordance with Art. 158(3) EPC

(43) Date of publication:
10.01.2007 Bulletin 2007/02

(51) Int Cl.:
C07D 263/32 (2006.01)     A61K 9/14 (2006.01)
A61K 31/421 (2006.01)     A61P 3/06 (2006.01)
A61P 3/10 (2006.01)

(21) Application number: 05734765.0

(22) Date of filing: 19.04.2005

(86) International application number:
PCT/JP2005/007763

(87) International publication number:
WO 2005/103017 (03.11.2005 Gazette 2005/44)

(84) Designated Contracting States:
AT BE BG CH CY CZ DE DK EE ES FI FR GB GR
HU IE IS IT LI LT LU MC NL PL PT RO SE SI SK TR

(30) Priority: 20.04.2004 JP 2004123582

(71) Applicant: ONO PHARMACEUTICAL CO., LTD.
Osaka-shi, Osaka 541-8526 (JP)

(72) Inventors:
• KUWABE, Shin-itsu
c/o Ono Pharmaceutical Co., Ltd.
Sakai-shi,
Fukui 913-8538 (JP)
• MASAOKA, Hideo,
c/o Ono Pharmaceutical Co., Ltd.
Sakai-shi,
Fukui 913-8538 (JP)
• ABE, Nobutaka,
c/o Ono Pharmaceutical Co., Ltd.
Osaka 618-8585 (JP)

(74) Representative: Henkel, Feiler & Hänzel
Patentanwälte
Maximiliansplatz 21
80333 München (DE)

(54) METHOD FOR INCREASING SPECIFIC SURFACE AREA OF SLIGHTLY SOLUBLE DRUG

(57) The preparation method of the powder of which dissolution rate improves is **characterized by** including of the process that slightly soluble drug having acidic group (e.g., 3-(5-{2-[5-methyl-2-(4-methylphenyl)-1,3-oxazol-4-yl]ethoxy}-3,4-dihydronaphthalen-1-yl)propanoic acid *etc.)* is made to metal salt solution and is neutralized to precipitate. By the method of the present invention, since a powder of which specific surface area increases and dissolution rate improves can be obtained, it is excellent as a preparation method of drug bulk

**Description**

Technical Field

The present invention relates to a powder of slightly soluble drug having an improved solubility and a method of increasing the specific surface area of a slightly soluble drug. More in detail, the present invention relates to a powder of 3-(5-{2-[5-methyl-2-(4-methylphenyl)-1,3-oxazol-4-yl]ethoxy}-3,4-dihydronaphthalen-1-yl)propanoic acid having an improved solubility and a method of increasing the specific surface area of a slightly soluble drug having acidic group is characterized by including the process that neutralizes a solution of a metal salt of a slightly soluble drug having acidic group to precipitate the drug.

Background Art

In formulation for oral administration, in general, a drug is dissolved in digestive tracts, absorbed, entries into systemic circulation blood and then achieves efficacy. However, since a slightly soluble drug has slow solution velocity, it is discharged outside the body without it being absorbed enough in vivo and then there are the problems that its bioavailability does not elevate. For example, 3-(5-{2-[5-methyl-2-(4-methylphenyl)-1,3-oxazol-4-yl]ethoxy}-3,4-dihydronaphthalen-1-yl)propanoic acid is described in WO02/051820 pamphlet. This compound is known to have peroxisome proliferator activated receptor (PPAR) agonistic effect and to be useful as a preventing and/or treating drug for diabetes and hyperlipemia *etc*. However, since this compound has low solubility, it has the problems for oral administration.
A lot of techniques for improving the solution velocity of a slightly soluble drug have been proposed. Feasible techniques among these are known to (1) a clathration technique using cyclodextrin, (2) a technique to form solid solution to become amorphia, (3) a microparticulation technique using grinder *etc*.
However, the technique (1) is hard to be said a general technique too much, because there are cases that suitable cyclodextrin can not be found out in some drugs since the size of a slightly soluble drug that is become clathration has to be suitable. In the technique (2), there are the problems that solubility of a drug temporarily increases when it is dispersed into water but after constant time course, a drug is deposited easy to lower solubility. In addition, since amorphia is originally in instable condition, there are the problems that it is susceptible to effect of light, heat, humidity *etc.*, or is over time easy to change stable crystal form. The technique (3) is the most general technique and the technique that makes average particle diameter below 1 micrometer has been also known (see JP H03-66613). However, in case of a drug is generally become microparticulation, aggregability and adhesive property of particle increase, particle adheres to grinder and grinding is easy to become difficult, therefore this technique has problems as industrial technique.

Disclosure of the Invention

Therefore, it is an object of the present invention that is to provide a slightly soluble drug having an improved solubility and a method to improve the solution velocity of a slightly soluble drug more easily and more effectively.
Considering the above-described problems, as a result of the inventors of the present invention made further investigation to surprisingly find out that the powder having increased specific surface and having improved the solution velocity was obtained by making the slightly soluble drug having acidic group (e.g. 3-(5-{2-[5-methyl-2-(4-methylphenyl)-1,3-oxazol-4-yl]ethoxy}-3,4-dihydronaphthalen-1-yl)propanoic acid (hereinafter abbreviated to the compound A)) into a solution of metal salt and neutralizing it to precipitate. In addition, the inventors of the present invention found out that the above-described compound A having the particular specific surface area had the dramatically improved solution velocity and it was excellent when it was used as bulk of drug, and completed the present invention.
That is, the present invention relates to

1. A pharmaceutical composition comprising of a powder of 3-(5-{2-[5-methyl-2-(4-methylphenyl)-1,3-oxazol-4-yl]ethoxy}-3,4-dihydronaphthalen-1-yl)propanoic acid, its specific surface area being adjusted to the range of 9 to 18 m$^2$/g,
2. The pharmaceutical composition according to the above 1, wherein a bulk density of the powder is in the range of 0.05 to 0.3 g/cm$^3$,
3. The pharmaceutical composition according to the above 1, wherein the time reaching dissolution rate 85% in dissolution test is within 30 minutes,
4. A pharmaceutical composition comprising of a powder of 3-(5-{2-[5-methyl-2-(4-methylphenyl)-1,3-oxazol-4-yl]ethoxy}-3,4-dihydronaphthalen-1-yl)propanoic acid having its specific surface area in the range of 9 to 18 m$^2$/g, being bulk density of its powder 0.05 to 0.3 g/cm$^3$ and being the time reaching dissolution rate 85% in dissolution test within 30 minutes,
5. A method of increasing specific surface area of a powder of 3-(5-{2-[5-methyl-2-(4-methylphenyl)-1,3-oxazol-4-yl]ethoxy}-3,4-dihydronaphthalen-1-yl)propanoic acid characterized by including a process for neutralizing a solution of metal salt of 3-(5-{2-[5-methyl-2-(4-methylphenyl)-1,3-oxazol-4-yl]ethoxy}-3,4-dihydronaphthalen-1-yl)propanoic acid to precipitate drug,

6. A process for preparing a powder of 3-(5-{2-[5-methyl-2-(4-methylphenyl)-1,3-oxazol-4-yl]ethoxy}-3,4-dihydronaphthalen-1-yl)propanoic acid having its specific surface area in the range of 9 to 18 $m^2/g$ characterized by including a process for neutralizing a solution of metal salt of 3-(5-{2-[5-methyl-2-(4-methylphenyl)-1,3-oxazol-4-yl]ethoxy}-3,4-dihydronaphthalen-1-yl)propanoic acid to precipitate drug,

7. The process for preparing according to the above 6, wherein metal salt is sodium salt,

8. The process for preparing according to the above 6, wherein in a process of neutralization, acid pouring into a mixed solution of metal salt of 3-(5-{2-[5-methyl-2-(4-methylphenyl)-1,3-oxazol-4-yl]ethoxy}-3,4-dihydronaphthalen-1-yl)propanoic acid in water and alcohol solvent,

9. A powder of 3-(5-{2-[5-methyl-2-(4-methylphenyl)-1,3-oxazol-4-yl]ethoxy}-3,4-dihydronaphthalen-1-yl)propanoic acid, its specific surface area being adjusted to the range of 9 to 18 $m^2/g$, and

10. The powder according to the above 9, which is prepared by the method according to the above 6.

A slightly soluble drug having acidic group includes, for example, 3-(5-{2-[5-methyl-2-(4-methylphenyl)-1,3-oxazol-4-yl]ethoxy}-3,4-dihydronaphthalen-1-yl)propanoic acid (compound A; the compound described in WO02/051820) and so on. It is not limited to this compound and it includes other slightly soluble drugs having acidic group.

A powder of 3-(5-{2-[5-methyl-2-(4-methylphenyl)-1,3-oxazol-4-yl]ethoxy}-3,4-dihydronaphthalen-1-yl)propanoic acid includes, for example, crystalline powder and amorphous powder and so on.

Metal salt includes, for example, alkali metal salt (e.g. potassium, sodium, lithium *etc.*), alkali earth metal salt (e.g. calcium, magnesium *etc.*) and so on. It is preferably alkali metal salt and more preferably sodium salt.

In the present invention, a method for neutralizing compounds includes, for example, the following methods.

1) A slightly soluble drug having acidic group is added into a mixed solution of water, alcohol solvent (e.g. methanol, alcohol, propanol, isopropanol *etc.*) and alkali (earth) metal hydroxide (e.g. potassium hydroxide, sodium hydroxide, lithium hydroxide, calcium hydroxide, magnesium hydroxide *etc.*), a mixture is dissolved with heat and then a solution of metal salt of slightly soluble drug having acidic group is prepared. After that, the above-described prepared solution is dropped into acid (e.g. concentrated hydrochloric acid, dilute hydrochloric acid aqueous solution *etc.*), a precipitate is separated by filtration, washed and dried by the law of the art and a powder having increased specific surface area can be obtained.

2) A slightly soluble drug having acidic group is added into a mixed solution of water, alcohol solvent (e.g. methanol, alcohol, propanol, isopropanol *etc.*) and alkali (earth) metal hydroxide (e.g. potassium hydroxide, sodium hydroxide, lithium hydroxide, calcium hydroxide, magnesium hydroxide *etc.*), a mixture is dissolved with heat and then a solution of metal salt of slightly soluble drug having acidic group is prepared. After that, the above-described prepared solution is dropped into acid (e.g. concentrated hydrochloric acid, dilute hydrochloric acid aqueous solution *etc.*), a precipitate is separated by filtration, washed and dried by the law of the art and a powder having increased specific surface area can be obtained.

In the above-described method for neutralizing, specific surface area of obtaining powder can be adjusted by adjusting temperature in a solution including metal salt of a slightly soluble drug having acidic group and by controlling ratio of water and alcohol solvent.

A process that adjusts specific surface area of a powder (e.g. crystal) of the compound A to the range of 9 to 18 $m^2/g$ includes, for example, preferably a process that the compound A is added into a mixed solution of water, alcohol solvent and alkali (earth) metal hydroxide, a mixture is dissolved with heat and then a solution of metal salt of the compound A is prepared, after that, the above-described prepared solution is dropped into acid, a precipitate is separated by filtration, washed and dried by the law of the art. As alcohol solvent, methanol or ethanol is preferred, ethanol is more preferred. As alkali (earth) metal hydroxide, potassium hydroxide or sodium hydroxide is preferred, sodium hydroxide is more preferred. As acid, hydrochloric acid is preferred. The temperature in solution when acid pours into the solution is preferably in the range of 35 to 55°C, and more preferably in the range of 45 to 55°C. The solvent ratio (capacity) of water and alcohol solvent is preferably water : alcohol solvent = 1.5 to 3.5 : 1, and more preferably water : alcohol solvent = 2.0 to 3.0 : 1.

A specific surface area of a powder of the compound A obtained by the above-described methods and the following Examples can be measured by known method, for example, multipoint BET method.

A specific surface area of a powder of the compound A is preferably in the range of about 9 to about 45 $m^2/g$, more preferably in the range of about 9 to about 30 $m^2/g$, further preferably in the range of about 9 to about 18 $m^2/g$, and particularly preferably in the range of about 9 to about 15 $m^2/g$. Especially, since the powder of the compound A having its specific surface area of about 9 to about 18 $m^2/g$ does not only have superior solubility, but also have superior filterability, the powder is very suitable for preparing on a industrial scale.

A bulk density of a powder of the compound A is preferably in the range of about 0.05 to about 0.3 g/cm$^3$, and more preferably in the range of about 0.05 to about 0.2 g/cm$^3$. A bulk density can be measured by known method. It includes, for example, the method that about 3 g sample is precisely weighed, it is put into dried 50 mL graduated cylinder (scale: by 0.1 mL) without consolidation, it reads to minimum unit of scale and value that the weight (g) of particulate is divided by the volume (cm$^3$) of particulate is assumed bulk density (g/cm$^3$), and method described in the following Examples and so on.

A tap density of a powder of the compound A is preferably in the range of about 0.1 to about 0.4 g/cm$^3$, and more preferably in the range of about 0.1 to about 0.3 g/cm$^3$. A tap density can be measured by known method. It includes, for example, the method that about 3 g sample is precisely weighed, it is put into dried 50 mL graduated cylinder (scale: by 0.1 mL) without consolidation, the graduated cylinder is set on tapping machine for tap density, then it is tapped for 3 minutes at 60 times/minute, final tap density is found, and value that the weight (g) of particulate is divided by final tap density (cm$^3$) of particulate is assumed tap density (g/cm$^3$), and method described in the following Examples and so on.

In the present invention, the dissolution rate in the dissolution test, according to dissolution test method 2 (Paddle method) of general test in the Japanese Pharmacopoeia, is the value that the method is performed on condition that 50 rounds per minute in pH6.8 McIlvaine buffer solution including 2.0% (w/v) sodium lauryl sulfate. More detail, it includes the method described in the following method or the method pursuant thereof and so on.

A powder of the compound A is preferably the powder of which time reaching dissolution rate 85% in dissolution test is within 30 minutes, more preferably within 20 minutes, and further preferably within 15 minutes.

When the pharmaceutical composition comprising of a powder of the compound A is administered, it is used in the form of internal solid or internal liquid agent for oral administration, injection, agent for external application, suppository for parenteral administration or the like. Preferable form includes internal solid agent for oral administration. Examples of the internal solid agent for oral administration include tablet, pill, capsule, powder, and pellet. Examples of the capsule include hard capsule, and soft capsule. In such an internal solid agent, one or more active materials are used in the form of preparation produced by an ordinary method singly or in admixture with a vehicle (e.g., lactose, mannitol, glucose, microcrystalline cellulose, starch *etc.*), binder (e.g., hydroxypropyl cellulose, hydroxypropyl methyl cellulose, polyvinyl pyrrolidone, magnesium metasilicoaluminate *etc.*), disintegrant (e.g., calcium fibrinoglycolate, low substituted hydroxypropylcellulose (L-HPC) *etc.),* glidant (e.g., magnesium stearate *etc.*), stabilizer, dissolution aid (e.g., glutamic acid, aspartic acid *etc.*) or the like. Additionally, if required, the solid agent may be coated with a coating agent (e.g., white sugar, hydroxypropyl methyl cellulose, gelatin, hydroxypropyl cellulose, hydroxypropyl methyl cellulose phthalate *etc.)* or two or more layers. Alternatively, the solid agent may be capsulized by an absorbable material such as gelatin.

Since a pharmaceutical composition comprising of a powder of the compound A has a PPAR (particularly, PPAR $\alpha$ and $\beta$) agonistic activity, it is useful for a hypoglycemic drug, a hypolipidemic drug and preventive and/or therapeutic drug for a PPAR mediated disease. PPAR mediated disease includes, for example, metabolic disorder (e.g., diabetes, obesity, metabolic syndrome, hyperlipemia (e.g., hypercholesterolemia, hypo-HDL (high-density lipoprotein)-cholesterolemia, hyper-LDL (low-density lipoprotein)-cholesterolemia, hypertriglyceridemia *etc.) etc.*), atherosclerosis, hypertension, cardiovascular disease, bulimia and so on.

Effect of the Invention:

According to the method of the present invention, since a powder of which specific surface area increased and of which solution velocity improved is obtained, it is very useful as a method for preparing bulk of drug.

Brief Description of the Drawing

Figure 1 represents the electron microgram (thousand fold and twenty thousand fold) of a powder of the compound A of which specific surface are of 42.24 m$^2$/g prepared in Adjustment Example 1.
Figure 2 represents the electron microgram (twenty thousand fold) of a powder of the compound A of which specific surface area of 20.6 m$^2$/g prepared in Adjustment Example 3.
Figure 3 represents the electron microgram (twenty thousand fold) of a powder of the compound A of which specific surface area of 12.51 m$^2$/g prepared in Adjustment Example 4.
Figure 4 represents the electron microgram (twenty thousand fold) of a powder of the compound A of which specific surface area of 5.46 m$^2$/g prepared in Adjustment Example 6.
Figure 5 represents a result of dissolution test using a powder of the compound A prepared in Adjustment Example 4 and 6.

Best Mode of the Carrying out the Invention

The present invention is explained below in detail based on Preparation Example, Adjustment Example of specific surface area and so on, however, the present invention is not limited thereto. The solvents in parentheses at chromatographic separations section and TLC section show the eluting or developing solvents and the ratios of the solvents used are indicated by volume.

Unless it especially describes, NMR data is 1H NMR data.

The solvents in parentheses indicated in NMR section show solvents used in determination.

The compound names used in the specification are named by using of ACD/Name (Trade mark, Advanced Chemistry Development Inc.) or ACD/Name batch (Trade mark, Advanced Chemistry Development Inc.) which is the computer program to name according to IUPAC rule, or according to IUPAC organic chemistry nomenclature

Preparation Example 1

Preparation of (2S)-4-methoxy-2-[(4-methylbenzoyl)amino]-4-oxobutanoic acid:

Under atmosphere of argon, a suspension of L-aspartic acid-β-methylester (100g) in tetrahydrofuran (1000mL) was dropped by N,O-bis(trimethylsilyl)acetamide (145.5g) at room temperature and stirred for an hour at 60°C. The reaction solution was cooled down to room temperature, was dropped by p-toluic acid chloride (110.9g) and N,N-diisopropylethylamine (92.3g) successively below 35°C and stirred for 2 hours at room temperature. The reaction solution was added by methanol (50mL) and stirred for an hour at room temperature. The reaction solution was concentrated and brown oil matter (600g) was obtained. The obtained oil matter was added by 5% methylamine aqueous solution (465g) and then extracted by *tert*-butyl methylether (400mL). The aqueous layer was acidified by 6mol/L hydrochloric acid (136mL) and extracted by ethyl acetate (300mL and 200mL). The combined organic layer was washed with saturated brine (250mL) and concentrated to give the title compound (184g) having the following physical data. The obtained compound was used in next reaction without more purification.

$^1$HNMR (400 MHz, CDCl$_3$) : δ 7.72 (d, J = 8.0 Hz, 2H), 7.36 (d, J = 7.2 Hz, 1H), 7.25 (d, J = 8.0 Hz, 2H), 5.09-5.04 (m, 1H), 3.74 (s, 3H), 3.17 (dd, J = 17.2, 4.4 Hz, 1H), 2.98 (dd, J = 17.2, 4.8 Hz, 1H), 2.41 (s, 3H).

Preparation Example

Preparation of methyl 3-[(4-methylbenzoyl)amino]-4-oxopentanoate:

A mixed solution of acetic anhydride (250mL), glacial acetic acid (15mL) and 4-dimethylaminopyridine (1.9g) was heated to 84°C, a solution of the compound (132g) prepared in Preparation Example 1 in pyridine (265mL) and ethyl acetate (530mL) added to the mixed solution and the mixture was stirred for an hour at 81 to 89°C. The reaction solution was cooled down to room temperature and then the solution was added by water (800mL) at temperature of 8 to 18°C in solution. The reaction solution was added by ethyl acetate (660mL) and the solution was separated. The aqueous layer was extracted by ethyl acetate (400mL). The combined organic layer was washed with 3mol/L hydrochloric acid (200mL, 2 times), mixed solution (saturated sodium carbonate aqueous solution 400mL, water 300mL) and saturated brine (200mL), successively and then concentrated to give the title compound (115.3g) having the following physical data.

$^1$HNMR (200 MHz, CDCl$_3$) : δ 7.72 (d, J = 8.4 Hz, 2H), 7.45 (d, J = 8.2 Hz, 1H), 7.26 (d, J = 8.4 Hz, 2H), 4.95 (ddd, J = 8.2, 4.6, 4.2 Hz, 1H), 3.70 (s, 3H), 3.11 (dd, J = 17.2, 4.2 Hz, 1H), 2.88 (dd, J = 17.2, 4.6 Hz, 1H), 2.41 (s, 3H), 2.29 (s, 3H).

Preparation Example 3

Preparation of methyl [5-methyl-2-(4-methylphenyl)-1,3-oxazol-4-yl]acetate:

A solution of the compound (61.6g) prepared in Preparation Example 2 in ethyl acetate (185mL) was added by acetic anhydrate (35.7g) and sulfuric acid (11.4g) successively at temperature of 15 to 19°C in the solution and the mixture was refluxed for an hour. The reaction mixture was cooled down, added by water (307mL), added by 4mol/L sodium hydroxide aqueous solution and the aqueous layer was adjusted to pH 7 to 8. The reaction solution was extracted by ethyl acetate (185mL). The aqueous layer was extracted by ethyl acetate (307mL). The combined organic layer was washed with saturated brine (154mL) and then concentrated to give the title compound (54.6g) having the following physical data.

$^1$HNMR (200 MHz, CDCl$_3$) : δ 7.91 (d, J = 8.2 Hz, 2H), 7.24 (d, J = 8.2 Hz, 2H), 3.73 (s, 3H), 3.60 (s, 2H), 2.39 (s, 3H), 2.36 (s, 3H).

Preparation Example 4

Preparation of 2-[5-methyl-2-(4-methylphenyl)-1,3-oxazol-4-yl]ethanol:

After substitution of argon, lithium aluminum hydride (826mg) was added by tetrahydrofuran (20mL) to make the suspension and then the suspension was cooled down to a temperature of 5°C in the solution, was slowly dropped by a solution of the compound (4.1g) prepared in Preparation Example 3 in tetrahydrofuran (25mL) and stirred for an hour at 10 to 20°C. The reaction solution was cooled down, slowly dropped by ethyl acetate (3mL) and the mixture was stirred for 10 minutes, after that, slowly added by methanol (5mL) and stirred for 30 minutes. The reaction mixture was added by a solution of sodium hydrogen tartaric acid (4.8g) in water (70mL) and extracted by ethyl acetate (35mL). The aqueous layer was extracted by ethyl acetate (35mL). The combined organic layer was washed with water (35mL, 2 times) and saturated brine (20mL) successively, dried by anhydrous magnesium sulfate and then concentrated to give the title compound (3.3g) having the following physical data.
TLC: Rf 0.18 (n-hexane : ethyl acetate = 2 : 1);
$^1$HNMR (200 MHz, CDCl$_3$) : δ 7.86 (d, J = 8.2 Hz, 2H), 7.23 (d, J = 8.2 Hz, 2H), 3.92 (dt, J = 6.0, 5.4 Hz, 2H), 3.31 (t, J = 6.0 Hz, 1H), 2.71 (t, J = 5.4 Hz, 2H), 2.39 (s, 3H), 2.33 (s, 3H).

Preparation Example 5

Preparation of 2-[5-methyl-2-(4-methylphenyl)-1,3-oxazol-4-yl]ethyl 4-methylbenzenesulfonate:

Under atmosphere of argon, the compound (500mg) prepared in Preparation Example 4 and p-toluenesulfonyl chloride (483mg) was added by acetonitrile (5mL) to make the suspension. The suspension was added by triethylamine (0.48mL) under the ice and the mixture was stirred for 20 hours at room temperature. The reaction solution was added by water (5mL) and stirred for 30 minutes at room temperature. The obtained solid was filtrated by suction filtration, the filtrate was washed with a mixed solution (acetonitrile 2mL and water 2mL) and then was dried under reduced pressure at 40°C overnight to give the title compound (732mg) having the following physical data.
TLC: Rf 0.69 (n-hexane : ethyl acetate = 1 : 1);
$^1$HNMR (200 MHz, CDCl$_3$) : δ 7.75 (d, J = 8.2 Hz, 2H), 7.66 (d, J = 8.2 Hz, 2H), 7.23 (d, J = 8.8 Hz, 2H), 7.18 (d, J = 8.2 Hz, 2H), 4.30 (t, J = 6.4 Hz, 2H), 2.81 (t, J = 6.4 Hz, 2H), 2.39 (s, 3H), 2.29 (s, 3H), 2.22 (s, 3H).

Preparation Example 6

Preparation of 5-oxo-5,6,7,8-tetrahydronaphthalen-1-yl pivalate

A suspension of 5-hydroxy-1-tetralone (160g) and triethylamine (151mL) in tetrahydrofuran (960mL) was dropped by pivaloyl chloride (130.8g) and the mixture was stirred for 3 hours at room temperature. The reaction solution was added by water (480mL) and the mixture was extracted by *tert*-butyl methylether (1280mL). The organic layer was washed with water (480mL, 2 times) and saturated brine (480mL) successively, dried over anhydrous magnesium sulfate and then concentrated. The residue was added by diethyl carbonate (100mL) and concentrated to give the title compound (299.6g) having the following physical data.
$^1$HNMR (200 MHz, CDCl$_3$,) : δ 7.73 (d, J = 7.8 Hz, 1H), 7.11 (t, J = 7.8 Hz, 1H), 6.97 (dd, J = 1.4, 7.8 Hz, 1H), 2.56 (t, J = 6.0 Hz, 2H), 2.42 (t, J = 6.0 Hz, 2H), 1.90 (m, 2H), 1.17 (s, 9H).

Preparation Example 7

Preparation of 5-bromo-7,8-dihydronaphthalene-1-yl pivalate:

A solution of the compound (20g) prepared in Preparation Example 6 in acetonitrile (100mL) was added by triphenyl phosphate (25.4mL) and pyridine (3.8g). The reaction solution was dropped by bromine (14.2g) and the mixture was stirred at room temperature overnight. The reaction solution was added by ethyl acetate (100mL) and saturated sodium thiosulfate aqueous solution (50mL) and the mixture was separated. The aqueous layer was extracted by ethyl acetate (50mL). The combined organic layer was washed with saturated brine (50mL) and then concentrated. The residue was purified by column chromatography on silica gel (n-hexane : ethyl acetate = from 25 : 1 to 15 : 1) to give the title compound (15.1g) having the following physical data.
$^1$HNMR (200 MHz, CDCl$_3$) : δ 7.44 (d, J = 7.6 Hz, 1H), 7.22 (t, J = 7.6 Hz, 1 H), 6.91 (d, J = 7.6 Hz, 1 H), 6.43 (t, J = 5 Hz, 1 H), 2.68 (t, J = 7.8 Hz, 2 H), 2.35(m, 2 H), 1.39 (s, 9H).

Preparation Example 8

Preparation of ethyl 3-iodopropanoate:

Ethyl 3-chloropropanoate (100g) and sodium iodide (274g) was suspended to acetonitrile (300mL) and the suspension was refluxed for 5 hours. The reaction solution was cooled down to room temperature, added by water (300mL) and toluene (275mL) and the mixture was separated. The aqueous layer was extracted by toluene (75mL). The combined organic layer was washed with saturated sodium thiosulfate aqueous solution (100mL), dried over anhydrous magnesium sulfate and then concentrated. The residue was added by tetrahydrofuran (200mL) and concentrated. The obtained residue was added by tetrahydrofuran (200mL) again and concentrated. The residue was dried overnight to give the title compound (150.1g) having the following physical data.
$^1$HNMR (400 MHz, CDCl$_3$) : δ 4.19 (q, J = 7.2 Hz, 2 H), 3.33 (t, J = 7.0 Hz, 2 H), 2.97 (t, J = 7.0 Hz, 2 H), 1.28 (t, J = 7.2 Hz, 3 H).

Preparation Example 9

Preparation of 5-(3-ethoxy-3-oxopropyl)-7,8-dihydronaphthalen-1-yl pivalate:

Under atmosphere of argon, zinc powder (2.5g) and copper bromide (0.58g) was added by acetonitrile (12mL) and the mixture was stirred at room temperature until green of copper bromide origin disappeared. The reaction solution was heated until 60°C, added by a solution of the compound (5.9g) prepared in Preparation Example 8 in acetonitrile (14mL) and the mixture was stirred for 2 hours at 60°C. The reaction solution was dropped by a solution of the compound (4.0g) prepared in Preparation Example 7, palladium acetate (15mg) and bis(2-diphenylphosphinophenyl) ether (DPEphos; 69.5mg) in acetonitrile (15mL) and the mixture was stirred at 80°C overnight. The reaction solution was cooled down to room temperature and then the insoluble matter was filtrated by suction filtration. The filtrate was added by 1mol/L hydrochloric acid (20mL) and *tert*-butyl methylether (60mL) and the mixture was separated. The aqueous layer was extracted by *tert*-butyl methylether (20mL). The combined organic layer was washed with saturated brine (20mL), dried over anhydrous magnesium sulfate and then concentrated. The part (1.0g) of the obtained residue (5.1g) was purified by column chromatography on silica gel (n-hexane : ethyl acetate = 5 : 1) to give the title compound (591 mg) having the following physical data.
$^1$HNMR (200 MHz, CDCl$_3$) : δ 1.25 (t, J = 7.05 Hz, 3H), 1.38 (s, 9H), 2.22 (m, 2H), 2.53 (m, 4H), 2.78 (m, 2H), 4.14 (q, J = 7.14 Hz, 2H), 5.90 (t, J = 4.58 Hz, 1H), 6.86 (dd, J = 7.51, 1.83 Hz, 1H), 7.16 (m, 2H).

Preparation Example 10

Preparation of ethyl 3-(5-hydroxy-3,4-dihydronaphthalen-1-yl)propanoate:

Under the ice, a solution of the compound (8.0g) prepared in Preparation Example 9 in ethanol (24mL) was added by a solution of sodium ethylate in 20% ethanol (13.2mL) and the mixture was stirred for 4 hours at room temperature. A solution of cooled citric acid (3.25g) in water (20mL) was dropped by the above-described reaction solution. The reaction solution was added by water (17.2mL) and ethyl acetate (20mL) and the mixture was separated. The aqueous layer was extracted by ethyl acetate (15mL, 2 times). The combined organic layer was washed with saturated brine (10mL), dried over anhydrous magnesium sulfate and then concentrated. The residue was purified by column chromatography on silica gel (n-hexane : ethyl acetate = from 25 : 1 to 10 : 1) to give the title compound (3.7g) having the following physical data.
$^1$HNMR (200 MHz, CDCl$_3$,) : δ 7.06 (t, J=7.87 Hz, 1H), 6.87 (d, J=7.51 Hz, 1H), 6.69 (d, J=7.87 Hz, 1H), 5.88 (t, J=4.49 Hz, 1H), 5.17 (s, 1H), 4.15 (q, J=7.14 Hz, 2H), 2.73 (m, 4H), 2.52 (m, 2H), 2.24 (m, 2H), 1.26 (t, J=7.14 Hz, 3H).

Preparation Example 11

Preparation of ethyl 3-(5-{2-[5-methyl-2-(4-methylphenyl)-1,3-oxazol-4-yl]ethoxy}-3,4-dihydronaphthalen-1-yl)propanoate:

A solution of the compound (5.4g) prepared in Preparation Example 5, the compound (3.0g) prepared in Preparation Example 10 and potassium phosphate (4.7g) in acetonitrile (30mL) was stirred for 4.5 hours at 80°C. The reaction solution was further added by the compound (0.9g) prepared in Preparation Example 5 and the mixture was stirred for 14.5 hours at same temperature. The reaction solution was added by water (30mL) at temperature of 70°C in the solution and then the mixture was cooled down for 30 minutes. The precipitated crystal was filtrated by suction filtration, the obtained crystal was washed with a mixed solution (acetonitrile 15mL and water 15mL), dried under reduced pressure for 10 hours at 40°C to give the title compound (5.4g) of yellow crystal having the following physical data.
$^1$HNMR (300 MHz, CDCl$_3$) : δ 7.85 (d, J = 8 Hz, 2H), 7.25 (d, J = 8 Hz, 2H),7.15 (dd, J = 8, 8 Hz, 1H), 6.90 (d, J = 8 Hz, 1H), 6.80 (d, J = 8 Hz, 1H), 5.85 (m, 1H),4.25 (t, J = 7 Hz, 2H), 4.15 (q, J = 10 Hz, 2H), 3.00 (t, J = 7 Hz, 2H), 2.80-2.65 (m, 4H), 2.50 (m, 2H), 2.40 (s, 3H), 2.38 (s, 3H), 2.20 (m, 2H), 1.25 (t, J = 10 Hz, 3H).

Preparation Example 12

3-(5-{2-[5-methyl-2-(4-methylphenyl)-1,3-oxazol-4-yl]ethoxy}-3,4-dihydronaphthalen-1-yl)propanoic acid (compound A):

The compound (2.0g) prepared in Preparation Example 11 was dissolved in a mixed solvent of methanol (5mL) and tetrahydrofuran (5mL) and the mixture was added by activated carbon (400mg) and 2mol/L sodium hydroxide aqueous solution (5.4mL) and stirred at temperature of about 60°C in the solution for 6 hours. The reaction solution was cooled down below 30°C and then activated carbon was removed by filtration, washed with a mixed solution of methanol (7mL) and tetrahydrofuran (7mL). The filtrate was set to temperature of 20 to 30°C, added by 2mol/L hydrochloric acid (5.4mL) and the mixture was stirred for 10 minutes at 20 to 30°C. Temperature in the obtained slurry was cooled down till 0 to 5°C and the slurry was stirred for 30 minutes and then the generated crystal was collected by filtration. The obtained crystal was washed with water (16mL) and then the crystalline powder (1.65g) of compound A of which specific surface area was 2.4 m$^2$/g by drying under reduced pressure at 40°C.
$^1$HNMR (200 MHz, DMSO-d$_6$) : δ 12.1 (br, s, 1H), 7.79 (d, J = 8.0 Hz, 2H), 7.29 (d, J = 8.0 Hz, 2H), 7.14 (t, J = 7.87 Hz, 1H), 6.88 (t, J = 8.15 Hz, 2H), 5.84 (t, J = 4.76 Hz, 1H), 4.19 (t, J = 6.32 Hz, 2H), 2.91 (t, J = 6.32 Hz, 2H), 2.57 (m, 4H), 2.36 (m, 2H), 2.34 (s, 3H), 2.33 (s, 3H), 2.09 (m, 2H).

Preparation Example 13

Preparation of 3-(5-{2-[5-methyl-2-(4-methylphenyl)-1,3-oxazol-4-yl]ethoxy}-3,4-dihydronaphthalen-1-yl)propanoic acid sodium salt:

A solution of the compound (5.0g) prepared in Preparation Example 11 in ethanol (5.0mL) was added by 2mol/L sodium hydroxide aqueous solution (6.5mL) and the mixture was stirred for an hour at 50°C. The reaction solution was added by *tert*-butyl methylether (125mL) and the mixture was cooled down by ice for 30 minutes. The precipitated crystal was filtrated by suction filtration and the obtained crystal was washed with a mixed solution (ethanol 30mL and *tert*-butyl methylether 30mL) and dried under reduced pressure for 10 hours at 40°C to give the title compound (4.6g) of white crystal.
$^1$HNMR (300 MHz, DMSO-d$_6$) : δ 7.79 (d, J = 8.4 Hz, 2H), 7.29 (d, J = 8.4 Hz, 2H), 7.13 (dd, J = 8.1, 8.1 Hz, 1H), 6.89 (d, J = 8.1 Hz, 1H), 6.86 (d, J = 8.1 Hz, 1H), 5.79 (dd, J = 4.5, 4.5 Hz, 1H), 4.18 (t, J = 6.3 Hz, 2H), 2.91 (t, J = 6.3 Hz, 2H), 2.60-2.47 (m, 4H), 2.34 (s, 3H), 2.33 (s, 3H), 2.12-1.96 (m, 4H).

Preparation Example 14

Preparation of 3-(5-{2-[5-methyl-2-(4-methylphenyl)-1,3-oxazol-4-yl]ethoxy}-3,4-dihydronaphtnalen-1-yl)propanoic acid potassium salt:

A solution of the compound (1.0g) prepared in Preparation Example 12 in ethanol (9.0mL) was added by 2mol/L potassium hydroxide aqueous solution (1.2mL) and the mixture was refluxed for 30 minutes. The reaction mixture was cooled down to room temperature and then added by *tert*-butyl methylether. The precipitated solid was collected by filtration to give the title compound (985mg) having the following physical data.
$^1$HNMR (300MHz, DMSO-d$_6$) : δ 7.84 (d, J = 8.7 Hz, 2H), 7.29 (d, J = 8.7 Hz, 2H), 7.13-7.08 (m, 1H), 6.98 (d, J = 8.1 Hz, 1H), 6.83 (d, J = 8.1 Hz, 1H), 5.89-5.86 (m, 1H), 4.24 (t, J = 6.6 Hz, 2H), 2.97 (t, J = 6.6 Hz, 2H), 2.72-2.58 (m, 4H), 2.38 (s, 3H), 2.35-2.30 (m, 5H), 2.13-2.05 (m, 2H).

Adjustment of a specific surface area of the compound A:

A specific surface area of the compound A was adjusted to give the crystalline powders with different specific surface area by the following method. As well, the measurement of specific surface area was performed by multipoint BET method.

Adjustment Example 1

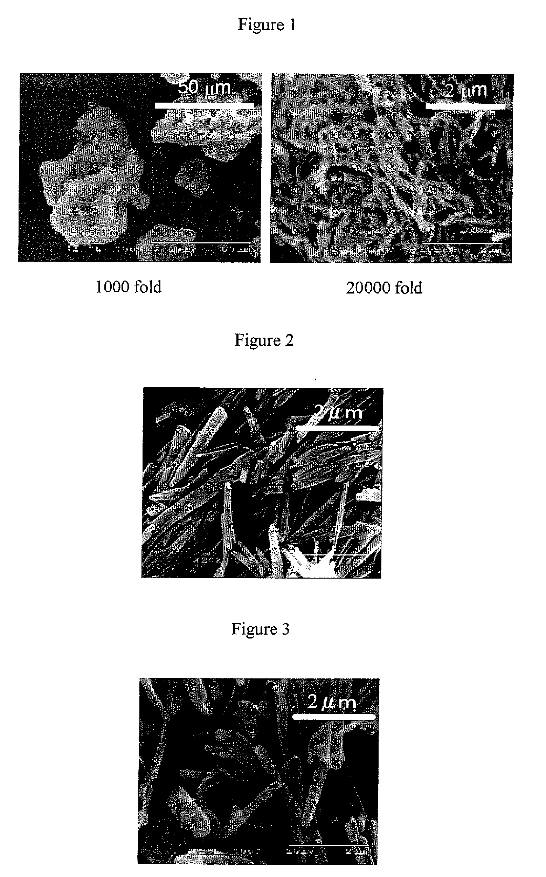

A mixture of ethanol (2L), water (2L) and sodium hydroxide (49.6g) was added by the compound A (475g) prepared in Preparation Example 12 and was heated to 40°C to be dissolved. Concentrated hydrochloric acid (142g) was diluted to water (15.6L) to prepare hydrochloric acid solution and cooled down to 10°C in the solution. Hydrochloric acid solution was dropped by the mixed solution prepared above, added by ethanol (500mL) and water (500mL) and the precipitate was filtrated. The precipitate was washed with water (30L), dried at 50°C under reduced pressure and then dried at 70°C to give the powder (389g) of the compound A having 42.24 $m^2$/g specific surface area as a aggregate. Figure 1 showed the electron microgram (1000 fold magnification and 20000 fold magnification)of this powder.

Adjustment Example 2

A mixture of ethanol (50mL), water (26.3mL) and 1mol/L sodium hydroxide solution (23.7mL) was added by the compound A (9.5g) prepared in Preparation Example 12, was heated to be dissolved, cooled down and added by 2mol/L hydrochloric acid (14mL). The mixed solution was added by water (100mL), stirred and the precipitate was filtrated. The precipitate was washed with water (30mL, 5 times) and then dried at 50°C under reduced pressure to give the powder (9.16g) of the compound A having 20.43 $m^2$/g specific surface area.

Adjustment Example 3

A mixed solvent of ethanol (175.5L) and water (175.5L) was added by the sodium salt of compound A (35kg) prepared in Preparation Example 13, heated to 30 - 50°C in the solution and dissolved. The temperature in this solution was cooled down till 30°C, was added by 2mol/L hydrochloric acid (43.8L) and then added by water (350L) and the mixture was stirred for 30 minutes. The precipitate was filtrated, was washed with water and dried at 50°C under reduced pressure to give the powder (30.7kg, non-crushing composition) of compound A having 19.6 $m^2$/g specific surface area. Additionally, this powder was jet mill crushed to give the powder (crushing composition) of compound A having 20.6 $m^2$/g specific surface area. Figure 2 showed the electron microgram (20000 fold magnification) of this powder (crushing composition).

Adjustment Example 4

A mixture of ethanol (12L), water (31.1L) and 10% sodium hydroxide solution (4.5kg) was added by the compound A (4.5kg) prepared in Preparation Example 12 and heated to 50 - 60°C in the solution to be dissolved. This solution was dropped by 2mol/L hydrochloric acid (6.87kg) with keeping 50°C in the solution, added by water (47L) of 50°C in the solution and stirred for 30 minutes at 50°C. The mixed solution was cooled down to 30°C in the solution. The precipitate was filtrated, washed with water and then dried at 50°C under reduced pressure to give the powder (4268g) of compound A having 12.51 $m^2$/g specific surface area. Figure 3 showed the electron microgram (20000 fold magnification) of this powder.

Adjustment Example 4(1)

By the same procedure as Adjustment Example 4 except keeping 60°C in the solution when 2mol/L hydrochloric acid dropped, the powder of compound A having 6.7 $m^2$/g specific surface area was obtained.

Adjustment Example 4(2)

By the same procedure as Adjustment Example 4 except keeping 40°C in the solution when 2mol/L hydrochloric acid dropped, the powder of compound A having 17.8 $m^2$/g specific surface area was obtained.

Adjustment Example 4(3)

By the same procedure as Adjustment Example 4, the powder (non-crushing composition) of compound A having 11.0 $m^2$/g specific surface area was obtained. Additionally, this powder was jet mill crushed to give the powder (crushing composition) of compound A having 12.7 $m^2$/g specific surface area.

Adjustment Example 4(4)

By the same procedure as Adjustment Example 4, the powder (non-crushing composition) of compound A

having 11.2 m$^2$/g specific surface area was obtained. Additionally, this powder was jet mill crushed to give the powder (crushing composition) of compound A having 13.4 m$^2$/g specific surface area.

Adjustment Example 5

A mixture of ethanol (50mL), water (26.3mL) and 1mol/L sodium hydroxide solution (23.7mL) was added by the compound A (9.5g) prepared in Preparation Example 12 and heated till 52°C in the solution to be dissolved. This mixed solution was added by 2mol/L hydrochloric acid (14mL) with keeping 52°C in the solution and then added by water (100mL) and the mixture was stirred at 52°C. The precipitate was filtrated. The precipitate was washed with water (30mL, 5times) and then dried at 50°C under reduced pressure to give the powder (9.5g) of compound A having 5.00 m$^2$/g.

Adjustment Example 6

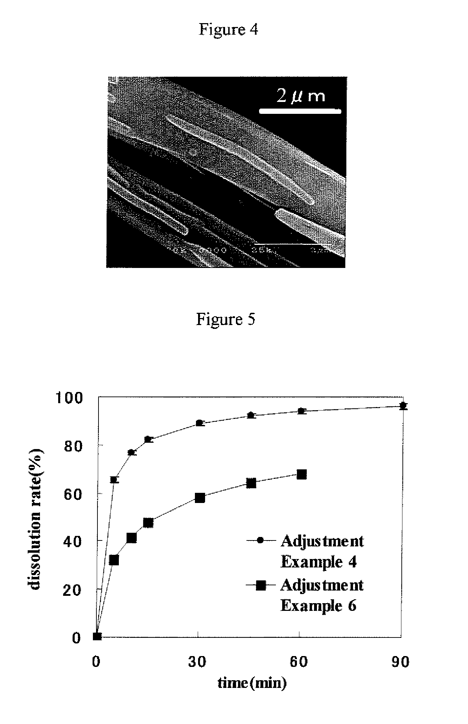

A mixed solvent of ethanol (110.3kg) and water (137.7kg) was added by the sodium salt of compound A (27.6kg) prepared in Preparation Example 13 and heated to 50°C in the solution to be dissolved. This solution was added by hydrochloric acid solution consisting of concentrated hydrochloric acid (7.04kg) and water (28.5kg) with keeping 50°C in the solution and then added by water (276L) and the mixture was stirred at 50°C for 1 hour. The mixed solution was cooled down until 0 - 5°C in the solution. The precipitate was filtrated, washed with water and then dried at 70°C under reduced pressure to give the powder (24.1kg) of compound A having 5.46 m$^2$/g specific surface area. Figure 4 showed the electron microgram (20000 fold magnification) of this powder.

Dissolution test:

The powder of compound A prepared in the above described Preparation Example 4 (specific surface area = 12.51 m$^2$/g) and Preparation Example 6 (specific surface area = 5.46 m$^2$/g) was sieved by 60 mesh sieve (250 $\mu$m) respectively and the sieved material was weighed out 30mg and then poured into the test solution. The dissolution test was performed under the below described condition of the dissolution test. However, the compound A was suspended with about 2mL test solution and then the compound A poured into the test solution. The eluate 10mL obtained at each sampling time was filtrated with membrane filter having 0.45 $\mu$m pore diameter. The first filtrate 2mL was removed and the rest of filtrate was as the solution of test material. The compound A prepared in Preparation Example 12 was precisely weighed out about 33.3mg and it was dissolved by adding methanol to adjust 100mL. Among this solution, 5mL was weighed out and it was added by test solution to adjust 50mL. It was made as the standard solution (0.0333mg/mL) that 100% of the compound A was dissolved. The absorbance of the solution of test material and standard solution in wavelength 278nm was measured by the measurement of ultraviolet visible absorbance respectively and according to the below formula, the dissolution rate was found.
The dissolution test condition:

Apparatus: The Japanese Pharmacopoeia, general test, dissolution test method 2 (Paddle method),
Test solution: McIlvaine buffer solution containing of 2.0 % (w/v) sodium lauryl sulfate, pH 6.8
Test solution volume: 900mL
Temperature: 37$\pm$0.5°C
Rotation speed: 50 rotations per minute
Sampling volume of eluate: 10mL
Sampling time: 5, 10, 15, 30, 45, 60 and 90 minutes.

$$\text{Concentration of the compound A in the solution of test material (mg/mL)} = \frac{W \times At}{As} \times \frac{1}{1000}$$

$$\text{The dissolution rate (\%)} = [Ct \times \{V - (t-1) \times v\} + \Sigma\, Cn \times v] \times \frac{100}{C}$$

W: The volume of compound A containing of standard solution (33.3mg)
t: The number of sampling
C: The volume of compound A poured into the test solution (30mg)

Ct: The concentration of compound A in the solution of test material at t times sampling

ΣCn: The sum of the concentration of compound A in the solution of test material from n=1 to t-1

At: The absorbance of the solution of test material

As: The average of absorbance of the solution of test material of repeated measurement at three times

V: The volume of the test solution (900mL)

v: The sampling volume of the eluate (10mL)

The result showed in Table 1 and Figure 5.

Table 1

| Time (minutes) | Dissolution rate (%) | |
|---|---|---|
| | Adjustment Example 4 | Adjustment Example 6 |
| 0 | 0.00 | 0.00 |
| 5 | 65.46 | 32.3 |
| 10 | 76.67 | 41.7 |
| 15 | 82.17 | 48.0 |
| 30 | 88.95 | 58.5 |
| 45 | 92.15 | 64.3 |
| 60 | 94.10 | 68.0 |
| 90 | 96.20 | - |

Result:

From the above described result, it was clear that the powder having increased specific surface area (Adjustment Example 4) was more elevated its dissolution rate than the powder having not increased specific surface area (Adjustment Example 6).

The measurement of bulk density:

About 3 g of compound A is precisely weighed, it is put into dried 50 mL graduated cylinder (scale: by 0.1 mL) without consolidation, it reads to minimum unit of scale. The value that the weight (g) of particulate is divided by the volume ($cm^3$) of particulate is assumed bulk density ($g/cm^3$). As a result, the bulk density of the powder of compound A prepared in Adjustment Example 4(3) was 0.18$g/cm^3$ (non-crushing composition) and 0.08g/cm3 (crushing composition). The bulk density of compound A prepared in Adjustment Example 4(4) was 0.18$g/cm^3$ (non-crushing composition) and 0.08$g/cm^3$ (crushing composition).

The measurement of tap density:

About 3 g of the powder of compound A is precisely weighed, it is put into dried 50 mL graduated cylinder (scale: by 0.1 mL) without consolidation, the graduated cylinder is set on tapping machine for tap density, then it is tapped for 3 minutes at 60 times/minute, final tap density is found. The value that the weight (g) of particulate is divided by final bulk density ($cm^3$) of particulate is assumed tap density ($g/cm^3$). As a result, the tap density of the powder of compound A prepared in Adjustment Example 4(3) was 0.28$g/cm^3$ (non-crushing composition) and 0.13$g/cm^3$ (crushing composition). The tap density of the powder of compound A prepared in Adjustment Example 4(4) was 0.23$g/cm^3$ (non-crushing composition) and 0.16$g/cm^3$ (crushing composition).[

Comparison of the filtration time:

The filtration time in process of Adjustment Example 3 and 4 was measured. However, filtration was performed by on like-for-like basis of suction filtration using Kiriyama funnel and filter paper having its diameter 40mm. As a result, the filtration time of Adjustment Example 3 (specific surface area of the powder of compound A is 19.6 $m^2/g$) was about 16 minutes than the time of Adjustment Example 4 (specific surface area of the powder of compound A is 12.51 $m^2/g$) was about 2.8 minutes. From these results, it was clear that the powder of compound A prepared in Example 4 was superior to filterability and was suitable to prepare on industrial scale.

Accelerated test:

1g of the powder of compound A was poured into the two-lay plastic bag and closed firmly with banding band. This was poured into miniature fiber dram and then this was preserved in the stability tester under the condition for 6 months, at 40°C, at 75% relative humidity. And then, the purity (quantitative value) and the degree of moisture (%) of the powder of compound A were measured. The measurement of the degree of moisture was performed by known method: the moisture vaporization method/ Karl Fisher electrometrical titration method. The measurement of purity was performed by high performance liquid chromatography (HPLC). The measurement condition showed below.
The absolute calibration method:

The procedure of measurement:

About 0.04mg/mL N,N-dimethylformamide/acetontirile (3/7) solution of reference material and measurement sample of compound A was prepared. In this case, the concentration of the solutions was precisely found. These solutions was set under the below test condition and the purity of measurement sample was calculated from the obtained area value of the peak.

The test condition:

The detector: ultraviolet absorptiometer (measurement wavelength: 278nm),
The column: YMC-Pack ODS-A-302 (inside diameter 4.6mm, length 15cm),
The mobile phase: acetonitrile/5mmol/L sodium acetate buffer (It was prepared to pH 4.5 using acetate.)/2-propanol mixture (5/4/1),
The flow: The retention time of the compound A was prepared to be about 12 minutes (about 1.0mL/min),
The column temperature: the constant temperature in the vicinity of 40°C.
The injection volume: 20 $\mu$L.

The measurement result of the powder of compound A prepared in Adjustment Example 3 (crushing composition, specific surface area: 20.6 m$^2$/g) and the powder of compound A prepared in Adjustment Example 4(4) (crushing composition: 13.4 m$^2$/g) showed the below table 2.

Table 2

| Retention time (month) | | 0 | 6 |
|---|---|---|---|
| Purity (%) | Adjustment Example 4(4) | 98.5 | 98.2 |
| | Adjustment Example 3 | 95.4 | 94.4 |
| Degree of moisture (%) | Adjustment Example 4(4) | 0.1 | 0.1 |
| | Adjustment Example 3 | 0.3 | 0.6 |

Result:

In the purity, that of the Adjustment Example 4(4) was decreased by only 0.3% than that of the Adjustment Example 3 was decreased by 1.0%. Additionally, in the degree of moisture, that of the Adjustment Example 4(4) had no change than that of the Adjustment Example 3 was increased by 0.3%. From these results, it was clear that the powder prepared in Example 4(4) was stable in long-term storage.

Industrial applicability

By the present method, since it is possible to prepare a powder of slightly soluble drug of which specific surface area increases and dissolution rate improves, it is excellent as a preparation method of drug bulk powder.

**Claims**

1. A pharmaceutical composition comprising a powder of 3-(5-{2-[5-methyl-2-(4-methylphenyl)-1,3-oxazol-4-yl]ethoxy}-3,4-dihydronaphthalen-1-yl)propanoic acid, its specific surface area being adjusted to the range of 9 to 18 $m^2/g$.

2. The pharmaceutical composition according to claim 1, wherein a bulk density of the powder is in the range of 0.05 to 0.3 $g/cm^3$.

3. The pharmaceutical composition according to claim 1, wherein the time reaching dissolution rate 85% in dissolution test is within 30 minutes.

4. A pharmaceutical composition comprising a powder of 3-(5-{2-[5-methyl-2-(4-methylphenyl)-1,3-oxazol-4-yl]ethoxy}-3,4-dihydronaphthalen-1-yl)propanoic acid having its specific surface area in the range of 9 to 18 $m^2/g$, being bulk density of its powder 0.05 to 0.3 $g/cm^3$ and being the time reaching dissolution rate 85% in dissolution test within 30 minutes.

5. A method of increasing specific surface area of a powder of 3-(5-{2-[5-methyl-2-(4-methylphenyl)-1,3-oxazol-4-yl]ethoxy}-3,4-dihydronaphthalen-1-yl)propanoic acid, which comprises neutralizing a solution of metal salt of 3-(5-{2-[5-methyl-2-(4-methylphenyl)-1,3-oxazol-4-yl]ethoxy}-3,4-dihydronaphthalen-1-yl)propanoic acid for precipitation.

6. A process for preparing a powder of 3-(5-{2-[5-methyl-2-(4-methylphenyl)-1,3-oxazol-4-yl]ethoxy}-3,4-dihydronaphthalen-1-yl)propanoic acid having its specific surface area in the range of 9 to 18 $m^2/g$, which comprises neutralizing a solution of metal salt of 3-(5-{2-[5-methyl-2-(4-methylphenyl)-1,3-oxazol-4-yl]ethoxy}-3,4-dihydronaphthalen-1-yl)propanoic acid for precipitation.

7. The process for preparing according to claim 6, wherein the metal salt is sodium salt.

8. The process for preparing according to claim 6, wherein in a process of neutralization, acid is poured into a mixed solution of metal salt of 3-(5-{2-[5-methyl-2-(4-methylphenyl)-1,3-oxazol-4-yl]ethoxy}-3,4-dihydronaphthalen-1-yl)propanoic acid in water and alcohol solvent.

9. A powder of 3-(5-{2-[5-methyl-2-(4-methylphenyl)-1,3-oxazol-4-yl]ethoxy}-3,4-dihydronaphthalen-1-yl)propanoic acid, its specific surface area being adjusted to the range of 9 to 18 $m^2/g$.

10. The powder according to claim 9, which is prepared by the method according to claim 6.

Figure 1

1000 fold                          20000 fold

Figure 2

Figure 3

Figure 4

Figure 5

## INTERNATIONAL SEARCH REPORT

| | International application No. |
|---|---|
| | PCT/JP2005/007763 |

**A. CLASSIFICATION OF SUBJECT MATTER**
Int.Cl⁷ C07D263/32, A61K9/14, 31/421, A61P3/06, 3/10

According to International Patent Classification (IPC) or to both national classification and IPC

**B. FIELDS SEARCHED**

Minimum documentation searched (classification system followed by classification symbols)
Int.Cl⁷ C07D263/32, A61K9/14, 31/421, A61P3/06, 3/10

Documentation searched other than minimum documentation to the extent that such documents are included in the fields searched

Electronic data base consulted during the international search (name of data base and, where practicable, search terms used)
REGISTRY(STN), CAplus(STN), MEDLINE(STN), EMBASE(STN), JSTPlus(JOIS)

**C. DOCUMENTS CONSIDERED TO BE RELEVANT**

| Category* | Citation of document, with indication, where appropriate, of the relevant passages | Relevant to claim No. |
|---|---|---|
| Y | WO 02/051820 A1 (Ono Pharmaceutical Co., Ltd.), 04 July, 2002 (04.07.02), Full text & CA 2432211 A  & EP 1354879 A1 & JP 3491635 B2  & BR 2001016526 A & NZ 526687 A  & NO 2003002895 A & ZA 2003004913 A  & JP 2004-123721 A & US 2004/138213 A1 | 1-10 |
| Y | Hiroshi NAKAGAWA et al., "Nanyosei Yakubutsu no Seizaika", Nippon Byoin Yakuzaishikai Zasshi, 1988, Vol.24, No.11, pages 1175 to 1182, particularly, page 1175, refer to the column of "2-1 increase of the surface area" | 1-10 |

☐ Further documents are listed in the continuation of Box C.     ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 05 July, 2005 (05.07.05) | 26 July, 2005 (26.07.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 02051820 A

- JP H0366613 B